# EUROPEAN PATENT APPLICATION

(11) **EP 2 251 003 A2**
(43) Date of publication of application: **17.11.2010**
(21) Application number: 10162084.7
(22) Date of filing: 06.05.2010
(51) Int. Cl.: A61K 9/00, A61K 47/32, A61K 45/06

(54) **Ophthalmic Compositions for the Re-epithelialization, Healing and Disinfection of the Eye Tissues**

(30) Priority: 13.05.2009 IT MI20090829
(71) Applicant: Bruschettini S.r.l., 16147 Genova (IT)
(72) Inventor: Bozzo Costa, Edoardo, 16136, GENOVA (IT)
(74) Representative: Minoja, Fabrizio

(57) **Abstract**

An ophthalmic composition comprising dexpanthenol, hexamidine diisethionate or chlorhexidine gluconate, polyvinyl alcohol and methylsulphonylmethane, performs a wound-healing, re-epithelialising and disinfectant action and, due to the viscoelastic hydrogel vehicle, prolongs the contact between the active substances and the ocular mucous membranes and increases their penetration, leading to the formation of a very thin, stable mucomimetic colloidal film which protects the eye tissues against bacterial infection for a long time, and facilitates their re-epithelialisation and healing in the event of lesions.

## Description

The present invention relates to ophthalmic compositions for the re-epithelialisation and disinfection of the eye tissues.

### BACKGROUND OF THE INVENTION

The human eye can be subjected to stresses (physical, mechanical, chemical, heat, etc.) which cause microlesions (e.g. caused by contact lenses, or small accidental traumatic lacerations of the cornea or conjunctiva) or larger and more important lesions.

Eye surgery also obviously causes lesions which are immediately sutured, but can benefit from topical administration of ophthalmic solutions containing re-epithelialising substances which accelerate the wound-healing processes, together with substances with bactericidal action which guarantee aseptic conditions of the eye tissues concerned.

In particular, many minor eye operations (including cataract removal) are often no longer performed in sterile hospital operating theatres, but in small clinics or doctors' surgeries, where the hygiene conditions are not always ideal.

Repeated administration during said operations of eyedrops that protect the eye tissues against infection and at the same time promote healing of the lesions can therefore be useful.

Dexpanthenol is the alcohol analogue of pantothenic acid, and is far more stable than the latter while maintaining equivalent biological activity, due to the reciprocal transformation. Said biological activity is closely correlated with participation in numerous metabolic processes essential for cell regeneration, such as synthesis of coenzyme A, a crucial stage in the metabolism of protein, fats and carbohydrates (Martindale Pharmacopoeia, current edition).

Its re-epithelialising (wound-healing) activity has been demonstrated in experiments *in vitro* which show activation of fibroblast proliferation in the cell, corresponding to a greater increase in aponeurosis *in vivo* (Egorov E.A. et al. New stimulation of corneal reparative regeneration. Vestn. Ophthalmol. 115 (6):13-15,1999). The effect of a 5% ophthalmic solution of dexpanthenol as adjuvant for corneal re-epithelialisation after experimentally induced lesions was tested *in vivo* in the same study on the eye of the rabbit. The results of said study clearly demonstrate that the extent and time of re-epithelialisation of the corneal endothelium are significantly stimulated by topical application of 5% dexpanthenol, and said effect is correlated with activation of the stromal fibroblast proliferation process and with faster migration of the corneal epithelium cells.

In the monograph reported in the German Federal Gazette (no. 24 of 5 Feb. 1993), it is reported that dexpanthenol is an effective adjuvant in healing lesions of the mucous membranes.

A study by Corner M. et al. (Cosmetic and Perfumery 1. 1974;89:35-39) reports data which demonstrate that pantothenic acid and dexpanthenol, applied topically, facilitate increased mitosis in lesions of the mucous membranes, due to an increase in cell division and consequent re-epithelialisation. The new epithelium that forms after topical application of dexpanthenol is stronger, due to the more compact structure of the deep layers of the epithelium.

WO 2003/049747 describes the use of panthenol and pantothenic acid with hyaluronic acid for the treatment of dry eye syndrome.

The toxicity of dexpanthenol is very low. Its oral LD50 is 6.25 g/Kg in the mouse and 3.0 g/Kg in the rabbit (German Federal Gazette, ibid.). According to a toxicology study (Liebert M.A. et al., J. Amer. College of Toxicology, 1987;6,(1): 139-162), the ocular tolerability of eyedrops containing dexpanthenol is excellent.

From the data reported above it can be concluded that dexpanthenol, applied topically to the eye, is useful for the reparatory regeneration of the corneal epithelium in the case of surgical operations on corneal microlesions in contact lens wearers, minor accidental lacerations of the cornea or conjunctiva, or eye damage caused by thermal or mechanical stress.

Hexamidine diisethionate is a diamidine with antibacterial and fungicidal action which has been used for decades, at the concentration of 0.100%, in ophthalmic preparations for topical treatment of minor eye infections (keratoconjunctivitis, conjunctivitis, blepharitis and dacryocystitis). Said substance, combined with polyhexanide hydrochloride in ophthalmic preparations, is used in the topical treatment of keratitis caused by Acanthamoeba.

The toxicological data reported in the literature indicate modest toxicity (oral LD50, mouse: 280 mg/kg; oral LD Lo, mouse 100 mg/kg; Toxicology and Applied Pharmacology; 34;271.1975; intraperit. LD50 mouse: 1060 mg/kg. British J. of Pharmacology; 37;140;1969. Ref: Registry of Toxic effects of chem. substances, Nat. Inst. Occupat. Safety and Health. Dept. Health and Human Serv. USA. 1979 ed). More recent studies report oral acute toxicity values, expressed as LD50, of 710-2500 mg/kg in the mouse and 750 mg/kg in the rat. Doses of 50 mg/kg/day administered orally to rats in a subchronic toxicity study did not show any toxic effect. Topical applications to the eye of the rabbit of aqueous solutions containing 0.10-0.11% of hexamidine diisethionate only caused slight, transient irritation, whereas no reaction was observed at the concentration of 0.050%.

Hexamidine diisethionate is not mutagenic, genotoxic or carcinogenic, and does not present any risk in terms of reproduction or foetal development.

Very few cases of sensitisation due to topical use of the substance have been reported in the literature (nine cases out of millions of subjects). As a precaution, the European Safety Assessment Cosmetic Ingredient Review Expert Panel (CIR) recommends that the concentration of 0.100% of hexamidine diisethionate should not be exceeded in preparations for topical use (eyedrops, dermatological creams, mouthwashes etc.) to avoid cases of irritation and sensitisation. (Final report on the safety assessment of Hexamidine Diisethionate. Int. J. Toxicol. 2007; 26 Suppl.: 79-88).

Chlorhexidine digluconate is a bisbiguanide with antiseptic and disinfectant action against a wide range of Gram-positive and Gram-negative bacteria, some types of mycobacteria, fungi and some viruses. It is present at different concentrations (0.02%, 0.05%, 0.5% and 1.00%) in topical preparations (aqueous sol. or creams) for disinfection of the skin, mucous membranes, wounds and burns. It is also present, combined with other substances, in numerous ophthalmic solutions which have been on the market for years.

The toxicity of chlorhexidine is considered modest (Extra Pharm. Martindale, current ed.). Topical treatments with chlorhexidine can give rise to sensitisation. Said symptoms, however, have been attributed to the use of topical solutions at high concentrations (0.500%-1.00%), and much more rarely with eyedrops at lower concentrations (0.005%).

Polyvinyl alcohol (PVA) is a synthetic non-ionic polymer which, in aqueous solution, has mucomimetic properties with a lubricant and emollient effect on the eye surface. When instilled into the eye in hydrogel form, its low surface tension increases the stability of the tear film, increasing the moisturisation of the cornea and reducing the surface tension of the tears.

The thin film formed by the PVA on the surface of the cornea and the conjunctiva provides adequate protection for the epithelium, and ensures lengthy contact between the disinfectant and re-epithelialising substance and the eye tissues.

The tolerability of PVA is excellent, as confirmed by the fact that it is present in the formulations of many artificial tears and eyedrops on the market.

Methylsulphonylmethane (MSM) is an organic compound containing sulphur, which is present in nature in numerous living organisms, including humans, in biologically active form.

It is used in ophthalmic preparations at the concentration of 5% for its antioxidant and nutrient properties, which are useful to the eye tissues. Its action involves increasing the permeability of the cell membranes and promoting penetration into the eye tissues of the active ingredients: dexpanthenol (wound-healing agent) and hexamidine diisethionate, or chlorhexidine gluconate (bactericides, disinfectants). MSM also performs a wound-healing action on the tissues, which is synergic with that performed by dexpanthenol.

The toxicity of MSM is very low, in both single and repeated doses. Horvath K. et al. (Food Chem. Toxicol, 2002, Oct. 40 (10): 1459-62) have demonstrated that doses of 1.5 g/kg/day administered for 90 days do not have any toxic effect. Magnusson BA et al (Food Chem Toxicol. 2007 Jun. 45 (6);977-84) have confirmed the absence of toxic effects at doses of 1 g/kg/day on fertility, gestation and neonatal development in the rat.

Studies of the metabolism of MSM demonstrate rapid absorption and rapid urinary elimination of said substance (Lin A et al., Toxicol. Lett. 2001, 123:169-177. Magnusson BA et al., J. Agric. Food Chem. 2007.55(3): 1033-38).

Moreover, a study by Saunders et al (Ophthalmology, 1992, 99: 1197-1200) confirms that the bactericidal activity of propamidine diisethionate, the homologue of hexamidine diisethionate, is boosted when it is carried in a solution containing dimethyl sulphoxide, which is the homologue of methylsulphonylmethane.

In a recent study, Zhang M et al (Drug Deliv. 2009, Jut.16 (5):243-48) demonstrated that MSM, as cell permeability activator, when administered to the cornea of the rabbit, promotes intraocular absorption of disodium edetate and other substances.

### DESCRIPTION OF THE INVENTION

The purpose of the present invention is to provide an ophthalmic composition with a re-epithelialising, wound-healing and bactericidal action which protects the eye tissues against infection, at the same time promoting healing of the lesions, and meets the following requirements:
- it avoids the use of bacteriostatics like antibiotics and chemical disinfectants, which are too aggressive and irritant, preferring substances which are not very toxic, well tolerated, including topically, have long been used in ophthalmic preparations, and whose safety is therefore well confirmed;
- it contains wound-healing substances whose re-epithelialising action has been scientifically demonstrated in published studies, with a proven action mechanism at epithelial cell level;
- it uses a vehicle perfectly tolerated by the eye tissues which provides lengthy contact with the active substances, thus ensuring an extremely effective bactericidal and wound-healing effect, and also contains an adjuvant that increases cell permeability and therefore facilitates penetration of the active ingredients into the eye tissues.

It has now been found that a composition containing dexpanthenol, hexamidine diisethionate or chlorhexidine gluconate, polyvinyl alcohol and methylsulphonylmethane meets said requirements.

According to the invention, dexpanthenol, a wound-healing and re-epithelialising agent, and hexamidine diisethionate (or chlorhexidine digluconate), bactericidal disinfectant agents, are incorporated in a viscoelastic hydrogel containing polyvinyl alcohol, which prolongs the contact between the active substances and the ocular mucous membranes, and methylsulphonylmethane, which increases the penetration of said substances into the eye tissues; the result is the formation of a very thin, stable mucomimetic colloidal film which protects the eye tissues against bacterial infection for a long period, and facilitates their re-epithelialisation and healing in the case of lesions.

The present invention therefore relates to an ophthalmic composition comprising:
a) dexpanthenol;
b) hexamidine diisethionate or chlorhexidine digluconate;
c) polyvinyl alcohol (PVA); and
d) methylsulphonylmethane (MSM).

As already stated, the ophthalmic composition according to the invention meets the ideal requirements for eyedrops with a re-epithelialising, wound-healing and disinfectant action, together with a synergic effect of the activities of the active ingredients, and fills a gap in topical eye treatment of the particular conditions indicated above, which require effective disinfection and re-epithelialisation of the eye tissues.

According to the invention, the composition in question is carried in a vehicle consisting of an aqueous hydrogel containing polyvinyl alcohol, whose mucomimetic characteristics ensure lengthy contact between the active substances and the eye tissues, and methylsulphonylmethane, an organic sulphur compound which aids the penetration of said substances into the tissues.

According to the present invention, the ophthalmic composition contains the various ingredients in the following percentage concentrations by weight, on the basis of the total weight of the composition:
a) dexpanthenol: 1.0% to 8.0%;
b) hexamidine diisethionate: 0.020% to 0.120%; or chlorhexidine digluconate: 0.001% to 0.050%;
c) polyvinyl alcohol (PVA): 0.800% to 1.600%; and
d) methylsulphonylmethane (MSM): 0.800% to 1.800%.

According to a preferred aspect of the invention, the ophthalmic composition will contain the various ingredients in the following percentage concentrations by weight, on the basis of the total weight of the composition:
a) dexpanthenol: 4.5% to 5%;
b) hexamidine diisethionate: 0.040% to 0.100; or chlorhexidine digluconate: 0.004% to 0.006%;
c) polyvinyl alcohol (PVA): 1.000% to 1.400%; and
d) methylsulphonylmethane (MSM): 1.00% to 1.400%.

The pH of the ophthalmic composition according to the present invention must be between 6.0 and 8.0, and preferably between 6.8 and 7.8. The pH can be adjusted with concentrated hydrochloric acid or a concentrated solution of sodium hydroxide, or by adding a buffer system, to maintain the pH within the required value range. Examples of suitable buffer systems are the combination of monobasic and dibasic sodium or potassium phosphate, buffer systems with sodium tetraborate, boric acid, sodium bicarbonate, citric acid citrate and the like.

The formulations according to the invention can also contain osmolarity correctors such as sodium chloride, to make the solution iso-osmolar with the lacrimal fluid if necessary.

The formulations according to the invention can also include other conventional additives commonly used in ophthalmic compositions, provided that they do not interfere with the activities of the active ingredients (re-epithelialising, wound-healing and bactericidal) or with the intracellular penetration activating effect or the effect of prolonged mucoadhesion to the eye tissues.

The formulations according to the invention can also include other compounds which have a complementary action or are otherwise useful for the purposes of the invention. Examples of said compounds are polyhexanide (polyhexamethylene biguanide) hydrochloride, used for its preservative and bactericidal action at a very low but effective concentration (0.0001 %), and disodium edetate, a known metal ion sequestering agent, which performs a stabilising and bacteriostatic action (conc. 0.010%).

According to the invention, one or two drops of the solutions of the compositions according to the invention can be instilled into the conjunctival sac of each eye every 2-3 hours, or more frequently if necessary.

The compositions according to the invention can be used for humans and for animals, including, but not limited to, dogs, cats, horses and cattle.

The compositions according to the invention can be formulated in the form of a moderately viscous colloidal solution, microemulsion, or a more viscous hydrogel.

A suitable formulation advantageously contains the following ingredients in the following percentages by weight:

| | |
|---|---|
| dexpanthenol | 5.000% |
| polyvinyl alcohol | 1.250% |
| methylsulphonylmethane | 1.250% |
| hexamidine diisethionate | 0.050% |
| (or: chlorhexidine digluconate) | 0.005% |
| polyhexanide hydrochloride | 0.0001 % |
| disodium edetate | 0.010% |
| dibasic sodium phosphate 12 H2O | 0.250% |
| monobasic potassium phosphate | 0.030% |
| purified water q.s. for | 100 g |

### FORMULATION EXAMPLES

### EXAMPLE 1 - Aqueous colloidal ophthalmic composition (hydrogel)

| Ingredient | Amount in grams |
|---|---|
| Purified water | 940.000 |
| Dexpanthenol | 50.000 |
| Polyvinyl alcohol | 12.500 |
| Methylsulphonylmethane | 12.500 |
| Hexamidine diisethionate | 0.500 |
| Polyhexanide hydrochloride | 0.001 |
| Disodium edetate | 0.100 |
| Dibasic sodium phosphate 12 H2O | 2.500 |
| Monobasic potassium phosphate | 0.300 |
| Total | 1018.401 |

The pH may optionally be adjusted with concentrated hydrochloric acid or a concentrated solution of sodium hydroxide.

### EXAMPLE 2 - Aqueous colloidal ophthalmic composition (hydrogel)

| Ingredient | | Amount in grams |
|---|---|---|
| Purified water | | 930.000 |
| Dexpanthenol | | 50.000 |
| Polyvinyl alcohol | | 12.500 |
| Methylsulphonylmethane | | 12.500 |
| Chlorhexidine digluconate | | 0.050 |
| Polyhexanide hydrochloride | | 0.001 |
| Disodium edetate | | 0.100 |
| Sodium tetraborate | | 3.000* |
| Boric acid | | 10.000* |
| Sodium chloride | | 0.050 |
| | Total | 1018.156 |

| | | |
|---|---|---|
| * or the amount required to adjust the pH to between 6.8 and 7.8 | | |

The composition described in Example 1 underwent osmolarity tests, and a value of approx. 380-400 m. Osmol/kg was found, which is slightly higher than, but perfectly compatible with, the mean value of the lacrimal fluid, confirming its excellent miscibility with said fluid and perfect tolerability.

The viscosity was approx. 1.6 - 2.0 cStokes, slightly higher than the mean values of the lacrimal fluid, because it is designed to ensure particularly useful mucoadhesion of the hydrogel to the surfaces of the ocular mucous membranes.

### STUDY OF ANTIMICROBIAL ACTIVITY

In addition to the factors described above, a specific study of the antimicrobial activity of the ophthalmic solution to which the present patent relates has been conducted, in particular on formulation No. 1) called Sterildex, which contains as bacteriostatics: hexamidine diisethionate, polyhexanide hydrochloride and sodium edetate, by comparison with the activity of other ophthalmic formulations on the market containing combinations of preservatives in common use.

Formulations of the ophthalmic solutions tested:
1) Sterildex batch 04: corresponds to formulation no. 1) of the present invention.
2) Bruviscreen batch 12): polyvinyl alcohol 1.250 g; sodium hyaluronan 0.010 g; glycerin 0.800 g; methyl hydroxybenzoate 0.030 g; propyl hydroxybenzoate 0.012 g; disodium edetate 0.030 g; N-methyl benzoquinoline methylsulphate 0.300 g; sodium chloride 0.600 g; dibasic sodium phosphate 0.090 g; monobasic potassium phosphate 0.020 g; distilled Hamamelis water 0.100 g; purified water q.s. for 100 ml.
3) Clarastill batch 1): carboxymethylcellulose 0.300 g; glycerin 1.000 g; methyl hydroxybenzoate 0.030 g; propyl hydroxybenzoate 0.012 g; disodium edetate 0.100 g; N-acetylcarnosine 1.000 g; sodium chloride 0.280 g; sodium tetraborate 0.108 g; potassium bicarbonate 0.048 g; purified water q.s. for 100 ml.
The test is conducted according to the procedure described in the European Pharmacopoeia, current ed.

### Method

Germ suspensions are prepared for inoculation at concentrations of approx. 10^5/10^6 germs/ml. The inoculation volume must not exceed 1% of the total volume of liquid to be examined.

The established volume of the suspension of micro-organisms (CFU/ml) is inoculated into test tubes (with triple control).

The suspension is thoroughly mixed to ensure complete distribution of the micro-organisms in the ophthalmic solutions tested, and incubated at +20°,+25°C, protected from light.

1 ml of the samples of ophthalmic solutions inoculated is taken up at time 0 and subsequently after 6-24 hours and after 7-14-28 days.

After each sampling, in accordance with the initial theoretical germ concentration and the expected reduction in CFU/ml, the solution is diluted 1:10 with saline solution, to obtain suspensions of germs at concentrations of approx. 100 CFU/plate. The dilutions obtained are filtered through Milliflex membranes filled with Sabouraud agar and CASO agar to eliminate the preservatives. The Sabouraud agar plates are incubated for 5 days at +20°+25°C and the CASO agar plates at +30°+35°C for 3 days. Blank tests are prepared in parallel with saline solution. Micro-organisms used:
- *Staphylococcus aureus* (bacterium)
- *Aspergillus niger* (fungus).

The results of the study of antimicrobial activity performed by the ophthalmic preparation of the present invention (Sterildex), by comparison with two other ophthalmic products (Bruviscreen and Clarastill), are summarised in the tables below.

**Table 1)**

| -----S. aureus----- | | | |
|---|---|---|---|
| Time in h and days | -Microbe conc. CFU/ml- | | |
| | -Sterildex- | -Bruviscreen- | -Clarastill- |
| T 0 | 300.000 | 1000000 | 500.000 |
| T 6 h\| | 8.000 | 180.000 | 6.000 |
| T 24 h | 10 | 1900 | 3.000 |
| T 7 days | 0.1 | 8 | 30 |
| T 28 days | 0.1 | 0.1 | 0.1 |

**Table 2)**

| -----A. niger----- | | | |
|---|---|---|---|
| Time in days | -Microbe conc. CFU/ml- | | |
| T 0 | 100.000 | 140.000 | 100.000 |
| T 7 days | 400 | 40 | 100 |
| T 14 days | 2 | 4 | 60 |
| T 28 days | 0.1 | 0.1 | 0.1 |
| | | | |

### Results

As shown by the data in Table 1), the product tested (Sterildex) performs an unexpected antibacterial activity on *S*. *aureus* which is much faster and more effective than that performed by the two comparator eyedrops.

The bacteria count (CFU/ml) was drastically reduced from an initial value (T0) of 300,000 to 8,000 after only 6 hours, 10 after 24 hours and nil after 7 days.

Said inactivation curve is unexpectedly far more rapid and significant than the Bruviscreen curve (from 1,000,000 CFU/ml at T0 to 180,000 after 6 hours, 1900 after 24 hours and 8 CFU/ml after 7 days), and the inactivation curve of Clarastill (from 500,000 CFU/ml at T0 to 6,000 after 6 hours, 3,000 after 24 hours and 30 after 7 days.).

The antimicrobial activity of the preparation in question against the fungus *A. niger* (table 2) is very effective, but comparable with that of the comparator products.

It can therefore be concluded that the ophthalmic preparation to which the present patent relates demonstrates an unexpectedly effective antimicrobial action against one of the most active and dangerous bacteria responsible for many frequent eye infections, *S*. *aureus.*

Said action is much more effective than that performed by the ordinary eyedrops on the market.

## Claims

1. Ophthalmic compositions containing:
a) dexpanthenol;
b) hexamidine diisethionate or chlorhexidine digluconate;
c) polyvinyl alcohol (PVA); and
d) methylsulphonylmethane (MSM).

2. Compositions as claimed in claim 1, containing the various ingredients in the following percentage concentrations by weight:
a) dexpanthenol: 1.0% to 8.0%;
b) hexamidine diisethionate: 0.020% to 0.120%; or chlorhexidine digluconate: 0.001% to 0.050%;
c) polyvinyl alcohol (PVA): 0.800% to 1.600%; and
d) methylsulphonylmethane (MSM): 0.800% to 1.800%.

3. Compositions as claimed in claim 2, containing the various ingredients in the following percentage concentrations by weight:
a) dexpanthenol: 4.5% to 5%;
b) hexamidine diisethionate: 0.040% to 0.100; or chlorhexidine digluconate: 0.004% to 0.006%;
c) polyvinyl alcohol (PVA): 1.000% to 1.400%; and
d) methylsulphonylmethane (MSM): 1.00% to 1.400%.

4. Compositions as claimed in the preceding claims, also including pH buffers based on monobasic and dibasic sodium and potassium phosphates; boric acid and sodium tetraborate, sodium or potassium bicarbonate, citric acid/citrates.

5. Compositions as claimed in the preceding claims, also including an osmolarity agent.

6. Compositions as claimed in claim 5, wherein the osmolarity agent is sodium chloride.

7. Compositions as claimed in the preceding claims, also including polyhexamethylene biguanide (polyhexanide hydrochloride) in amounts ranging between 0.00005% and 0.0001% on the weight of the composition.

8. Compositions as claimed in the preceding claims, also including disodium edetate, in amounts ranging between 0.005% and 0.100% on the weight of the composition.
